# EUROPEAN PATENT SPECIFICATION

(11) **EP 2 047 161 B1**
(45) Date of publication and mention of the grant of the patent: **11.12.2013**
(21) Application number: 07813462.4
(22) Date of filing: 27.07.2007
(51) Int. Cl.: A61M 39/16, A61M 39/04, A61M 39/26

(54) **VASCULAR ACCESS DEVICE NON-ADHERING MEMBRANES**
NICHT HAFTENDE MEMBRANEN FÜR EIN GEFÄSSZUGANGSGERÄT
DISPOSITIF D'ACCÈS VASCULAIRE NON ADHÉRENT AUX MEMBRANES

(30) Priority: 28.07.2006 US 820718 P; 26.07.2007 US 829013
(43) Date of publication of application: 15.04.2009
(73) Proprietor: Becton, Dickinson and Company, Franklin Lakes, NJ 07417 (US)
(72) Inventor: HARDING, Weston F., Lehi, Utah 84043 (US); MCKINNON, Austin Jason, Herriman, Utah 84065 (US); OU-YANG, David, Woodbury, Minnesota 55125 (US); GUO, Lantao, Draper, Utah 84020 (US)
(74) Representative: von Kreisler Selting Werner
(86) International application number: PCT/US2007/074578
(87) International publication number: WO 2008/014448

(56) References cited:
- WO-A1-99/32168
- WO-A1-99/61093
- US-A- 4 211 214
- US-A- 4 673 393
- US-A- 6 045 539
- US-A1- 2005 123 727
- US-A1- 2007 012 893
- US-B2- 6 866 656

## Description

### BACKGROUND OF THE INVENTION

The present disclosure relates to infusion therapy with vascular access devices. Infusion therapy is one of the most common health care procedures. Hospitalized, home care, and other patients receive fluids, pharmaceuticals, and blood products via a vascular access device inserted into the vascular system. Infusion therapy may be used to treat an infection, provide anesthesia or analgesia, provide nutritional support, treat cancerous growths, maintain blood pressure and heart rhythm, or many other clinically significant uses.

Infusion therapy is facilitated by a vascular access device. The vascular access device may access a patient's peripheral or central vasculature. The vascular access device may be indwelling for short term (days), moderate term (weeks), or long term (months to years). The vascular access device may be used for continuous infusion therapy or for intermittent therapy.

A common vascular access device is a plastic catheter that is inserted into a patient's vein. The catheter length may vary from a few centimeters for peripheral access to many centimeters for central access. The catheter may be inserted transcutaneously or may be surgically implanted beneath the patient's skin. The catheter, or any other vascular access device attached thereto, may have a single lumen or multiple lumens for infusion of many fluids simultaneously.

The proximal end of the vascular access device commonly includes a Luer adapter to which other medical devices may be attached. For example, an administration set may be attached to a vascular access device at one end and an intravenous (IV) bag at the other. The administration set is a fluid conduit for the continuous infusion of fluids and pharmaceuticals. Commonly, an IV access device is a vascular access device that may be attached to another vascular access device, closes or seals the vascular access device, and allows for intermittent infusion or injection of fluids and pharmaceuticals. An IV access device may include a housing and a septum for closing the system. The septum may be opened with a blunt cannula or a male Luer of a medical device.

Complications associated with infusion therapy may cause significant morbidity and even mortality. One significant complication is catheter related blood stream infection (CRBSI). An estimate of 250,000 - 400,000 cases of central venous catheter (CVC) associated BSIs occur annually in US hospitals. Attributable mortality is an estimated 32% - 25% for each infection and a cost to the health care system of $25,000 - $56,000 per episode.

Vascular access device infection resulting in CRBSIs may be caused by failure to regularly clean the device, a non-sterile insertion technique, or by pathogens entering the fluid flow path through either end of the path subsequent to catheter insertion. Studies have shown the risk of CRBSI increases with catheter indwelling periods. When a vascular access device is contaminated, pathogens adhere to the vascular access device, colonize, and form a biofilm. The biofilm is resistant to most biocidal agents and provides a replenishing source for pathogens to enter a patient's bloodstream and cause a BSI. Thus, what are needed are systems, devices, and methods to reduce the risk and occurrence of CRBSIs.
A medical device of the type defined in the first part of claim 1 is disclosed in WO 99/61093. This medical device comprises a vascular access device having a body and a septum. The septum comprises an antimicrobial agent within in an elastomer body. This antimicrobial agent provides an antimicrobial barrier associated with the elastormer, such as by coating or by molding. This assures that a comprehensive barrier is provided at all surfaces potentially contacting a separate extravascular device, such as a syringe. This assures that a barrier is provided at all surfaces potentially contacting a luer tip during insertion into the medical device.
WO 99/32168 describes a material that causes a continual supply of an antimicrobial agent and/or a lubricious agent to a surface of a medical device.
US 6 045 539 describes a sterile medical injection port, which comprises a hollow cylindrical housing, a pad of absorbent porous material disposed inside a bore, and a frangible sealed capsule disposed inside the bore and containing an antiseptic material.
US 2007/0012893 A1 discloses an elastomeric gland provided for a luer activating device and comprising a lubricant and/or wetting agent.

### BRIEF SUMMARY OF THE INVENTION

It is an object of the present invention to provide a vascular access device wherein the rate at which lubricant is exuded to specific surfaces of the vascular access device can be controlled.
The vascular access device of the invention is defined by claim 1.

The present invention has been developed in response to problems and needs in the art that have not yet been fully resolved by currently available vascular access systems, devices, and methods. Thus, these systems, devices, and methods are developed to reduce the risk and occurrence of CRBSIs by providing a membrane on, in, or integrated or compounded with the body of a vascular access device that prevents or discourages one or more pathogens from adhering to the surface. By discouraging pathogen adhesion, the non-adhering membranous surface prevents or limits pathogen colonization and proliferation into a biofilm and/or harmful culture. [0008] A medical device may include a vascular access device having a body and a membrane of the body. The membrane communicates with a pathogenic environment, and the membrane discourages adhesion of a pathogen to the membrane in order to repress pathogenic activity. The membrane may include one or more pores through which an antimicrobial fluid may be transferred from the vascular access device, through the pores, and into the fluid path of the vascular access device. [0009] The pores may be canals, the vascular access device may further include a reservoir in communication with the canals, and an antimicrobial agent and/or an oil or lubricant may be housed or stored within the reservoir. An antimicrobial agent or lubricant may be transferred to a surface of the vascular access device through the membrane as the vascular access device is accessed by a separate device.

The vascular access device may also be connected to a source of vacuum that communicates with the membrane. The membrane may include one or more pores, and the source of vacuum may pull one or more pathogens into one or more of the pores. As a pathogen is pulled into a pore, the membrane may shear the pathogen. [0011] The membrane may also include pores that house biocompatible gas bubbles, and each of the pores may be separated by a minimal surface area of the membrane. The surface area between two pores of the membrane may be less than the surface area of the attaching surface of a pathogen. The width of each of the pores may be at least twice the diameter of a pathogen.

These and other features and advantages of the present invention may be incorporated into certain embodiments of the invention and will become more fully apparent from the following description and appended claims, or may be learned by the practice of the invention as set forth hereinafter. The present invention does not require that all the advantageous features and all the advantages described herein be incorporated into every embodiment of the invention.

### BRIEF DESCRIPTION OF THE SEVERAL VIEWS OF THE DRAWINGS

In order that the manner in which the above-recited and other features and advantages of the invention are obtained will be readily understood, a more particular description of the invention briefly described above will be rendered by reference to specific embodiments thereof which are illustrated in the appended drawings. These drawings depict only typical embodiments of the invention and are not therefore to be considered to limit the scope of the invention.

Figure 1 is a perspective view of an extravascular system connected to the vascular system of a patient.

Figure 2 is a cross section view of a vascular access device with a porous septum.

Figure 3 is a cross section view of a vascular access device with a septum having a reservoir and channels.

Figure 4 is a side view of a vascular access device connected to a cross section view of a vacuum source.

Figure 5 is a partial cross section view of the vascular access device of Figure 4.

Figure 6 is a close-up, cross section view of a porous layer of the vascular access device of Figure 4.

Figure 7 is a close-up, cross section view of a porous layer of the vascular access device of Figure 4 with fragmented pathogens.

Figure 8 is a cross section view of a vascular access device having a gaseous membrane.

Figure 9 is a close-up, cross section view of the gaseous membrane of Figure 8.

### DETAILED DESCRIPTION OF THE INVENTION

The presently preferred embodiments of the present invention will be best understood by reference to the drawings, wherein like reference numbers indicate identical or functionally similar elements. It will be readily understood that the components of the present invention, as generally described and illustrated in the figures herein, could be arranged and designed in a wide variety of different configurations. Thus, the following more detailed description, as represented in the figures, is not intended to limit the scope of the invention as claimed, but is merely representative of presently preferred embodiments of the invention.

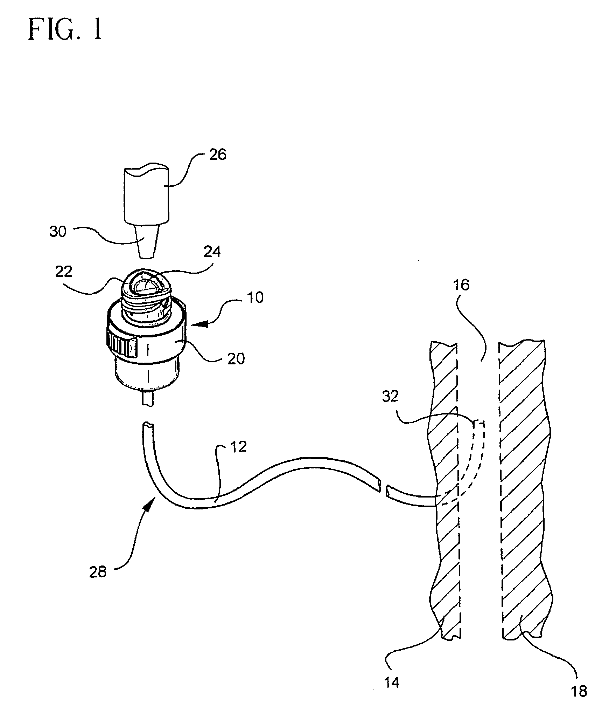

Referring now to Figure 1, a vascular access device (also referred to as an extravascular device, intravenous access device, access port, and/or any device attached to or functioning with an extravascular system) 10 is used to introduce a substance via a catheter 12 across the skin 14 and into a blood vessel 16 of a patient 18. The vascular access device 10 includes a body 20 with a lumen and a septum 22 placed within the lumen. The septum 22 has a slit 24 through which a separate extravascular device 26, such as a syringe, may introduce a substance into the vascular access device 10.

The device 10 also includes a membrane (discussed with reference to the figures below) integrated or compounded with, in, and/or on the body 20 of the device 10, an extravascular system 28, and/or septum 22. The membrane discourages, inhibits, prevents, or otherwise limits a pathogen from adhering to the membrane. By discouraging pathogen adhesion, the non-adhering membrane represses the pathogen by preventing or limiting pathogen colonization and proliferation into a biofilm and/or harmful culture. The membrane represses at least one pathogen to decrease the incidence of blood stream infections in patients to whom the vascular access device 10 or any other device on an extravascular system 28 is attached.

As described throughout this specification, pathogens include any agent that causes or facilitates a disease, infects, or otherwise harms or has the potential to harm a patient or host if received into the vascular system of that patient or host. A pathogen includes a pathogen, bacterium, parasite, microbe, biofilm, fungus, virus, protein feeding a pathogen, protozoan, and/or other harmful microorganisms and/or agents and products thereof. The membrane discourages a pathogen from adhering and/or represses pathogenic activity to prevent the proliferation, growth, or organization of a harmful biofilm by any one or combination of the following actions: removing, dislodging, repelling, resisting, detaching, loosening, unbinding, unfastening, releasing, separating, dividing, disconnecting, and/or freeing from a pathogen from a surface of the device 10 and/or any other similar process or action.

A pathogen may enter the device 10 or system 28 in any of a number of ways. For example, a pathogen may reside within the device 10 or system 28 prior to first use. A pathogen may also be introduced into the device 10 from the external surface of the device, the external surface of a separate device 26, and/or the surrounding environment when a structure such as a tip 30 of the separate device 26 is inserted into the device 10 through the slit 24 of the septum 22. A pathogen may be introduced within fluid that is infused into the system from a separate device 26. Finally, a pathogen may be introduced from a blood vessel 16 into the system 28 by entering through the end 32 of the catheter 12 during a blood draw or a period of blood reflux when the device 10 is in use. The membrane may thus be integrated, compounded, and/or placed in or on any surface, structure, or body of the entry, junctions, and/or fluid path of the system 28 in order to discourage pathogen adhesion and repress pathogenic activity, as desired.

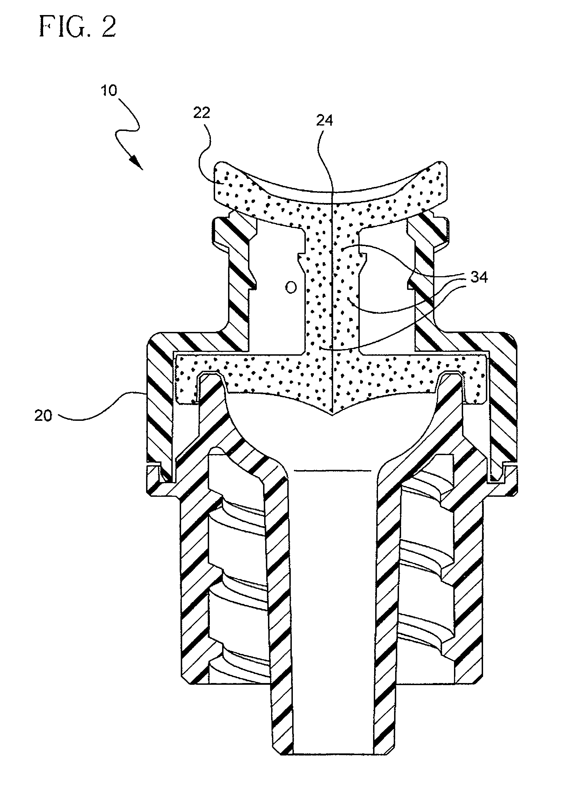

Referring now to Figure 2, a vascular access device 10 includes a body 20 and a septum 22 residing on an inner surface of the body 20. The septum 22 and/or the body 20 are examples of membranes that communicate with a pathogenic environment and discourage adhesion of a pathogen to the membrane in order to repress the activity of the pathogen. The septum 22, for example, includes multiple pores 34 through which an oil or other lubricant is transferred to an exterior surface of the septum 22, such as the surface of the slit 24 of the septum 22. The oil or lubricant may also include an antimicrobial agent or other fluid that may be transferred across the septum 22 membrane through the pores 34. The antimicrobial agent may be any agent capable of repressing the activity of a pathogen, including any of the antimicrobial agents listed in the following Table 1.

The material of the septum 22 is preferably a silicone capable of bleeding or otherwise eluding a lubricant through its pores. The material of the septum 22 may be regulated or otherwise coated with a material capable of limiting the porosity of the material. Thus, the rate at which lubricant is exuded to a pathogenic surface of the septum 22 can be controlled to provide an environment that is optimal for discouraging adhesion of a pathogen to the surface. According to the invention a surface of the septum 22 that is mechanically attached or otherwise connected to the body 20 includes a material which limits or eliminates the porosity of the material on those surfaces. Thus, at the mechanical connection between the septum 22 and the body 20, the septum 22 will not be lubricated, preventing any unwanted slipping between the septum 22 and the body 20.

**TABLE 1**

| Technology/Company | Antimicrobial Mechanism of Action | Active Ingredient |
|---|---|---|
| Alexidine | Bisbiguanide/Antiseptic | Alexidine |
| AMERICAL (Merodine) | Halogen/Antiseptic | lodine |
| Angiotech Pharmaceuticals | Antimicrobial/Antineoplastic | 5-Flurouracil |
| Apacidar (SGA) | Metals & Salts | Silver |
| Arglaes (Giltech) | Metals & Salts | Silver |
| Arrow Howes CHG and AgSD | Bisbiguanide/Antiseptic + antibiotic | Chlorhexidine and Silver Sulfadiazine |
| Bactifree | Metal & Salts | Silver |
| Bacterin | Metal | Silver Hydrogel |
| HASF PVP-1 Dusted Gloves BD | Halogen/Antiseptic | Iodine |
| Baxter American Edwards | Antiseptic and Anticoagulant | Benzalkonium Chlorine complexed Heparin |
| Benzalkonium Chloride | Quaternary Ammonium/Antiseptic | Benzalkonium Chloride |
| Benzethonium Chloride | Quaternary Ammonium/Antiseptic | Benzethonium Chloride |
| Bioshield (CATO Research) | Halogen/Antiseptic | Iodine |
| BisBAL | Metal, mercury | Bismuth and 2,3 dimercaptopropanol *a.k.a*.dimercaprol, or British anti-lewisite |
| CATO Research (Bioshield) | Halogen/Antiseptic | Iodine |
| Chlorhexidine (and its salts) | Bisbiguanide/Antiseptic | Chlorhexidine |
| Ciprofloxacin TDMAC Complex BD | Antibiotic | Ciprofloxacin |
| Cooke | TDMAC bound Antibiotic | Any antibiotic |
| Cosmocil | Bisbiguanide/Antiseptic | Cosmocil |
| Cyclodextrin | Nonstick surface | Cyclodextrin |
| Daltex | Bisbiguanide/Antiseptic | Chlorhexidine and Silver Sulfadiazine |
| Dicloxacillin TDMAC Complex BD | Antibiotic | Dicloxacillin |
| EDTA, EGTA | Calcium Chelator | EDTA, EGTA |
| Epiguard (Iodine) | Halogen/Antiseptic | Iodine |
| Epitope (Iodine) | Halogen/Antiseptic | Iodine |
| ExOxEmis | Oxidative enzymes | Mycloperoxidase and Eosinophil Peroxidase |
| Fusidic Acid TDMAC Complex BD | Antibiotic | Fusidic Acid |
| Gamma A Technologies | Specific Antibodies | Specific Antibodies |
| Giltech | Metal & Salts | Silver |
| Glyzine | Metals & Salts | Zinc |
| Gold | Metal & Salts | Gold |
| Healthshield | Metal & Salts | |
| Heparin-Benzalkonium Chloride | Antimicrobial Antithrombogenic | |
| Hexyl Bromide | Metals & Salts | Hexyl Bromide |
| Implemed (Ag/Pt) | Metal & Salts | Silver/Platinum |
| Intelligent Biocides | Metals & Salts | Silver |
| Iodine | HalogenAntiseptic | Iodine |
| Iodine Tincture | Halogen/Antiseptic | Iodine |
| Irgasan | Phenolic/Antiseptic | Triclosan |
| Johnson-Matthey | Metal | Silver |
| Kinetic Concepts | Metals & Salts | Silver |
| Luther Medical | Antibiotic | Polymyxin B |
| Lysozyme | Enzymatic Antibiotic | |
| Mediflex Chlorhexidine Gluconate Tincture | Bisbiguanide/Antiseptic | Chlorhexidine/Isopropanol |
| Merodine | Halogen/Antiscptic | Iodine |
| Microban | Antiseptic Polymer | Triclosan |
| Microbia | Antibiotic | "Natural" polypeptides |
| MicroFre | Metal & Salts | |
| Minocycline Rifampin | Antibiotic | Minocycline Rifampin |
| Minocycline-EDTA | Antibiotic | Minocycline EDTA |
| Morton Bloom | Cidal Lipids | Free fatty acids |
| Novacal | Neutrophil Cidal Factors | Oxidative Enzymes |
| Octenidine | Bisbiguanide/Antiseptic | Octenidine |
| Oligon (Implemed Ag/Pt) | Metal & Salts | Silver/Platinum |
| Olin Chemicals | Metal & Salts | Zinc |
| Omacide | Metal & Salts | Zinc |
| Omni Medical | Heterologous Antibodies | Antibodies |
| Orthophenyl phenol (Lysol) | Phenolic/Antiseptic | Orthophenyl phenol |
| Phosphorus | Antimicrobial Polymer | Phosphorus |
| Polymyxin B (Luther) | Antibiotic | |
| PVP-I (Iodine) | Halogen/Antiseptic | Iodine |
| Quorem Sciences | Cell-signalling | Peptides |
| Rifampin | Antibiotic | Rifampin |
| Sangi Group America | Metal & Salts | Silver |
| SGA | Metal Salts | Silver |
| Silver Chloride | Metal & Salts | Silver |
| Sliver Nitrate | Metal & Salts | Silver |
| Silver Oxide | Metal & Salts | Silver |
| Silver Palladium | Metal & Salts | Silver |
| Spi-Argent | Metal & Salts | Silver |
| Spire | Metal & Salts | Silver |
| Surfacine | Metal & Salts | Silver |
| TCC (Triclocarban) | Phenolic/Antiseptic | Triclocarban |
| TCS (Triclosan) | Phenolic/Antiseptic | Triclosan |
| TDMAC | Antibiotics | Cephazolin, Cipro., Clindamycin, Dicloxacillin, Fusidic Acid, Oxacillin, Rifampin |
| Triclocarban | Phenolic/Antiseptic | Triclocarban |
| Triclosan | Phenolic/Antiscptic | Triclosan |
| Vancomycin | Antibiotic | Vancomycin |
| Vancomycin-Heparin | Antibiotic | Vancomycin-Heparin |
| Vibax | Phenolic/Antiseptic | Triclosan |
| Vitaphore CHG coating | Bisbiguanide/Antiseptic | Chlorhexidine |
| Vitaphore Silver Cuff | Metal & Salts | Silver |
| Zinc | Metal & Salts | Zinc |
| Zinc Omadine | Metal & Salts | Zinc |

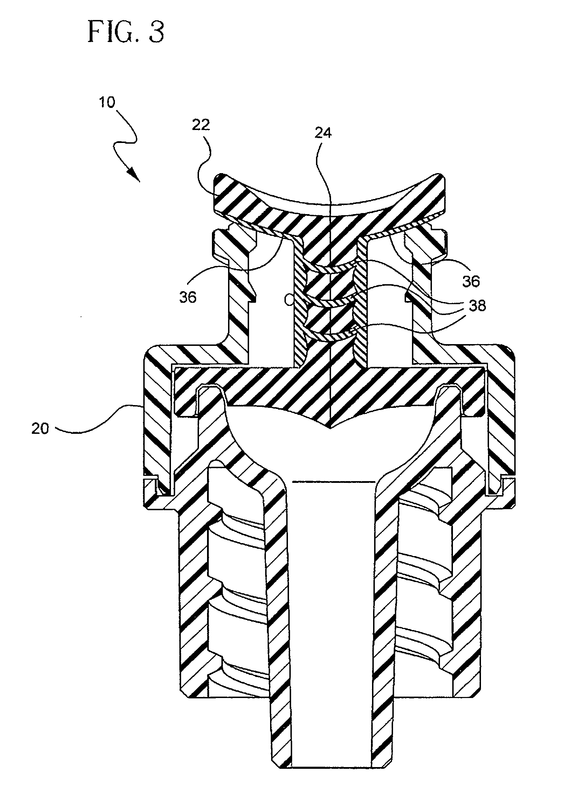

Referring now to Figure 3, a vascular access device 10 includes a body 20 and a septum 22 housed on the inner surface of the body 20. The septum 22 is a membrane including at least one reservoir 36 and multiple channels 38 in communication with the reservoir 36. The channels 38 function as pores similar to the pores 34 of Figure 2. The channels are capable of transferring an antimicrobial agent, such as the agents set forth in Table 1, across the membrane of the septum 22 to a surface of the vascular access device 10. As the vascular access device 10 is accessed, the antimicrobial agent is transferred from the reservoir 36 through the channels 38 to the inner surface of the septum 22.

For example, the vascular access device 10 may be accessed by a separate device 26, such that the tip 30 of a separate device 26 (Figure 1) is inserted into the slit 24 of the septum 22. As the tip 30 is inserted through the slit 24, the walls of the septum 22 will be forced outward toward the body 20 of the device 10. As the walls are forced outward, the reservoir 36 will be compressed, forcing the antimicrobial agent through each of the channels 38 and onto the surface of the slit 24. Thus, every time the device 10 is accessed, the pressure change within the reservoir 36 caused by the geometrical changes of the septum 22 causes the antimicrobial agent and/or any oil or lubricant residing therein to squeeze, move, spill, or otherwise be transferred into the main path of the slit 24 from the channels 38.

The embodiments described with reference to Figures 2 and 3 thus describe two alternate embodiments capable of providing a membrane that discourages a pathogen from adhering to a surface of a vascular access device. By providing oils or other lubricants and providing antimicrobial agents on a surface of the device 10 through a membrane, a pathogen that would otherwise be likely to attach to such surface and reside thereon, will be either killed or unable to attach to the surface as a result of its modified characteristics.

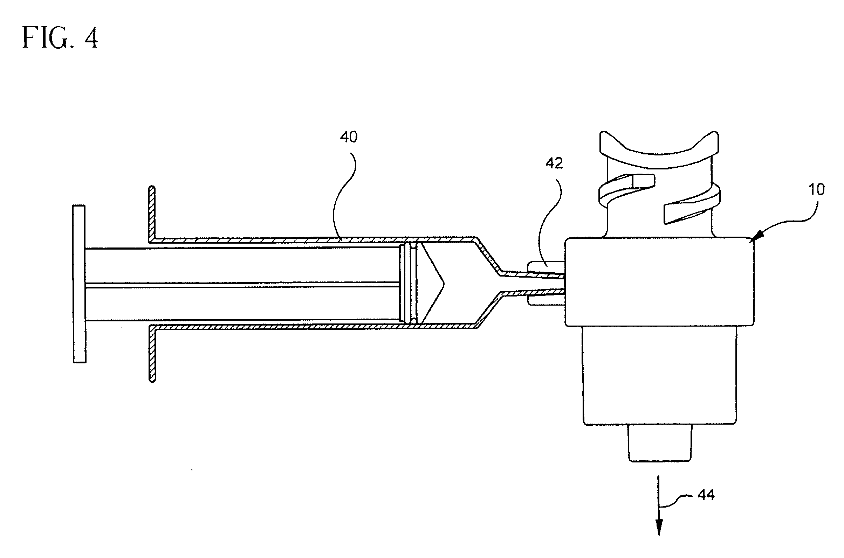

Referring now to Figure 4, a vascular access device 10 may be coupled with a vacuum source 40, such as a syringe, in order to pull a pathogen from the interior of the device 10 through one or more pores of the device 10 and into the vacuum source 40. The vacuum source 40 accesses the vascular access device 10 through a port 42 of the device 10.

In use, the device 10 may be clamped at location 44 downstream of the device 10, and the vacuum source 40 may then pull fluid from the device 10 through the port 42 and into the vacuum source 40. By clamping the device 10 downstream at location 44, an operator can avoid any unwanted reflux of fluid from the location 44 downstream up into the device 10 and ultimately into the vacuum source 40.

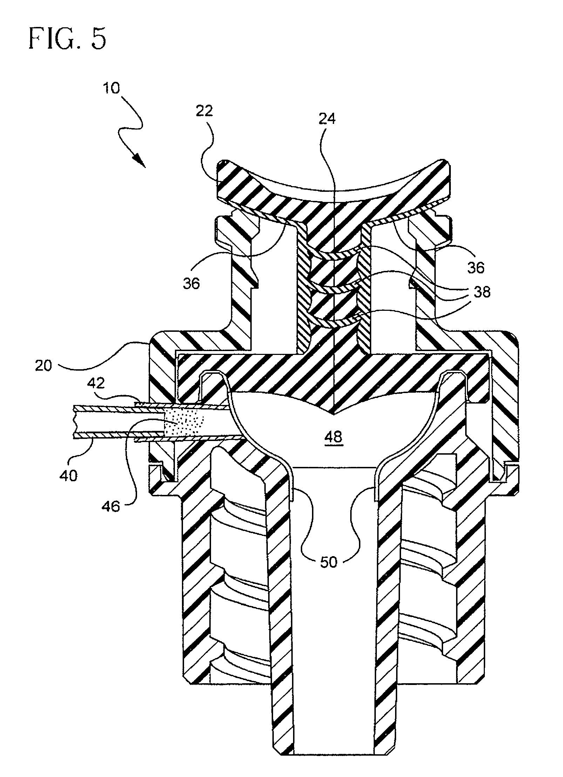

Referring now to Figure 5, a partial cross section view of the device 10 of Figure 4 is shown. The cross section reveals the port 42 providing a fluid exit to pathogens 46 that are removed from the device 10. The pathogens 46, such as bacteria, are transferred from the interior chamber 48 of the device 10, across a membrane of multiple pores 50, through the access port 42, and into the vacuum source 40.

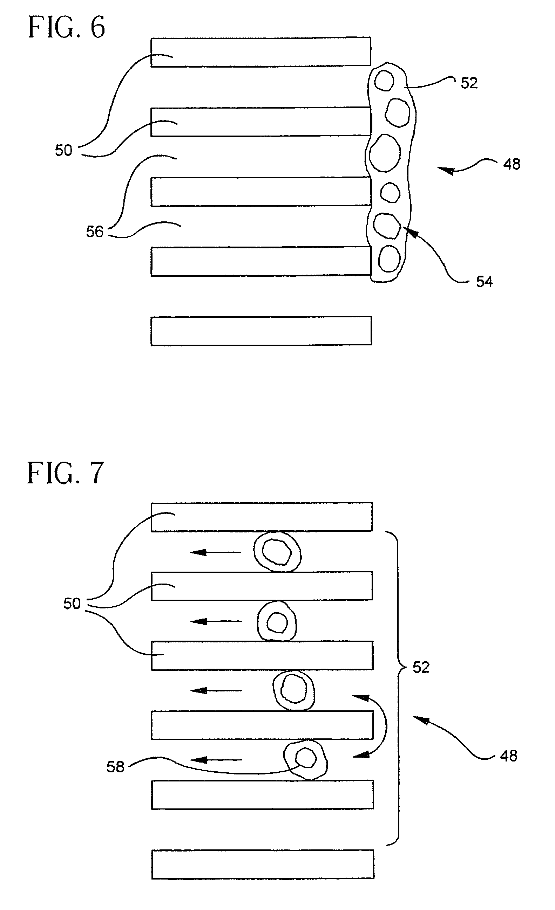

Referring now to Figure 6, the porous layer 50 of the vascular access device 10 of Figures 4 and 5 is shown in close-up, cross section view. The porous layer 50 reveals multiple pores that arc smaller than a bacterial biofilm 52 that has started to form on the interior surface 54 of the porous layer 50. The biofilm 52 is located on the interior surface 54, which is in turn located within the interior chamber 48 of the vascular access device 10. Each of the individual pores 56 of the porous layers 50 are preferably smaller than the neighboring pathogenic structure, the biofilm 52.

Referring now to Figure 7, the porous layer 50 of Figure 6 is shown in similar close-up, cross section view with the biofilm 52 broken into smaller pathogenic fragments 58. The pressure caused by vacuum source 40 has caused the biofilm 52 to break up, or to be sheared, and forced into separate channels or pores 56. The vacuum source 40 thus pulls through a backside of the device 10, through the access port 42, causing the biofilm 52 to break up into individual fragments 58 and be removed from the interior chamber 48, which is part of the fluid path of the device 10 to a patient.

The material forming the walls of the porous layer 50, the size and shape of the pores 56, and the size and shape of the walls of the porous layer 50 may be adjusted as preferred in order to provide a variety of embodiments capable of shearing a pathogen, pulling a pathogen and/or a portion of a pathogen into at least one pore, and/or discouraging adhesion of a pathogen to a membrane of a vascular access device. For example, the ends of the walls of the porous layer 50 at interior surface 54 may be pointed in order to provide more of a cutting surface capable of shearing, puncturing, or otherwise separating the biofilm 52 and/or a single pathogen cell when the vacuum source 40 exerts vacuum force through the pores 56 of the porous layer 50. Further, since many bacteria are approximately one micron in diameter, the diameter of the individual pores 56 may be less than one micron in diameter in order to encourage the dissection of a bacterium as it enters into one or more pores 56 under the influence of vacuum pressure.

Alternatively or additionally, the diameter of the individual pores 56 may be slightly larger than one micron, providing access to only a single cell pathogenic, and thus encouraging the single cell to remain in a live state and be removed from the biofilm 52, thus separating it from other neighboring bacterial cells. In its live state, the bacterial cell can later be analyzed to determine the characteristics of the pathogen that was residing within the device 10. Based on those results, an operator may administer appropriate treatment to the device 10 and/or the patient to which the device 10 is attached.

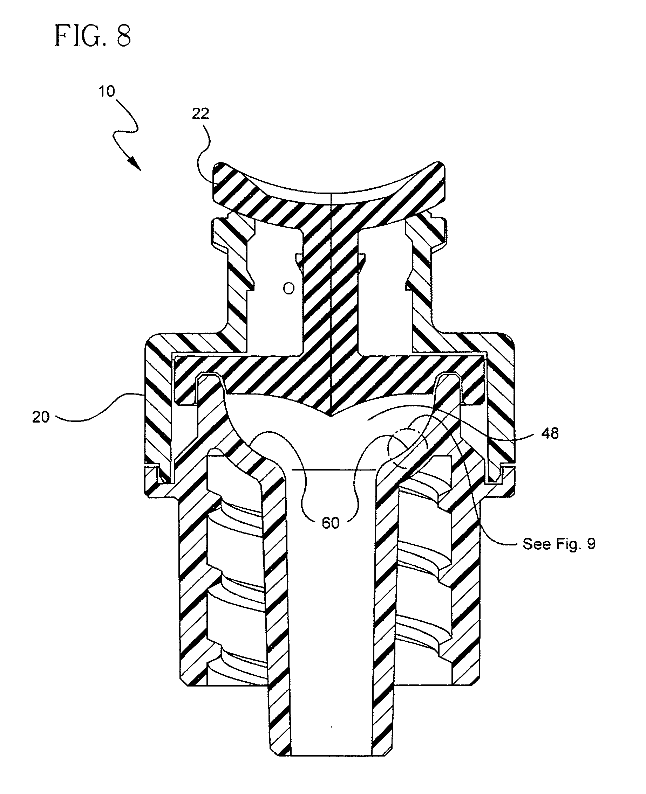

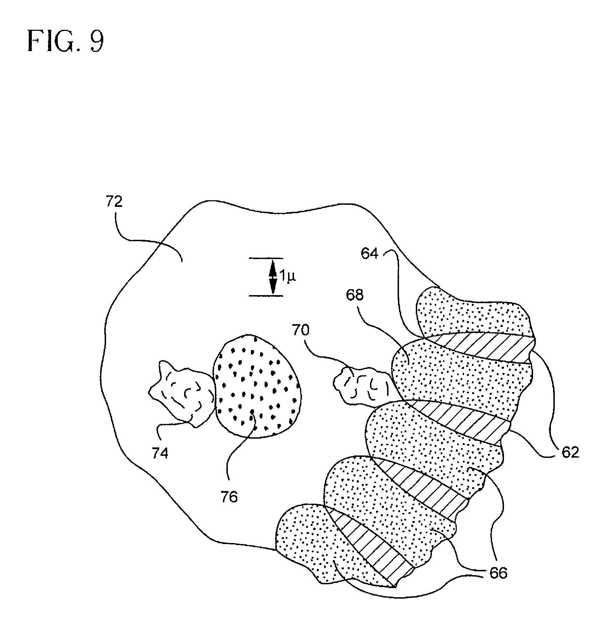

Referring now to Figure 8, a vascular access device 10 includes a membrane 60 located near the interior chamber 48 of the device 10. A close-up, cross section view of the membrane 60 is shown and further described with reference to Figure 9.

Referring now to Figure 9, the membrane 60 of the vascular access device 10 described with reference to Figure 8 is shown in close-up, cross section view. The membrane 60 is formed of multiple sequential structures 62, each with a minimal surface area at its tip 64. The structures 62 are separated by pores 66 between each of the structures 62. The pores 66 house and/or emit a biocompatible gas, capable of traveling through the pores 66, and forming the large majority of the barrier of the membrane 60. The gas 68 housed within each pore 66 is preferably a biocompatible gas capable of being redissolved in the lungs of a patient.

As shown in Figure 9, the surface area on the tip 64 of a structure 62, which is between two pores 66, is less than the surface area of the total attaching surface of a pathogen 70. Further, the width of an individual pore 66 may be at least twice the diameter of a pathogen 70. Depending upon the cohesive properties of the fluid 72 housed within the interior chamber 48, each of the gas bubbles formed within the pores 66 will form a dome of varying dimensions arching from the tip 64 of one structure 62 to the tip 64 of a neighboring structure 62. In this manner, the gas 68 of the pore 66 forms the majority of the barrier of the membrane 60, thus providing little to no mechanical surface to which the pathogen 70 may attach.

As the gas 68 travels through the pores 66 and into the interior chamber 48, the gas bubbles 68 will force any pathogen 70 that has attached to a tip 64 to be removed from the tip 64 and into the fluid 72. A pathogen 74 is thus shown having been removed from a tip 64 under the influence of a gas bubble 76. The pathogen 74 is removed from the surface of the membrane 60 before the pathogen 74 is able to colonate or otherwise develop, organize, or proliferate in order to form a harmful biofilm that would cause infection, injury, or other harm to a patient.

The gas bubbles 68, as mentioned earlier, may be at least twice the diameter of the diameter of a pathogen. For example, a pathogen having a one micron diameter may be removed by a gas bubble having a 2 to 3 micron diameter. The gas bubbles 68 may originate from any source of gas, either through a gas line attached to the vascular access device, or through cells neighboring the membrane 60. The cells neighboring the membrane 60 may include any living cell, chemical reaction, electrochemical reaction, or any other process capable of generating gas as a byproduct.

The embodiment described with reference to Figures 8 and 9 thus provides a membrane 60 capable of providing minimal surface area to which a pathogen may attach. By providing a membrane that is primarity gaseous, the pathogen will either bounce off or slide past the majority of the membrane and will only attach, if at all, at certain structural tips of the membrane. After the pathogen has attached to these tips, it will be too distant from a neighboring tip in order to combine and grow with another pathogenic friendly substance on a neighboring tip. Further, as gas is emitted through the pores of the membrane 60, the pathogens are forced from the tips into the interior chamber 48 of fluid path, further directing the pathogens, in their harmless state, ultimately into the patient.

The speed at which gas is transferred through the pores of the membrane 60 may be adjusted depending on the type of gas that is used, the type of pathogen that is likely to be present within the interior chamber 48, the type of treatment being administered to a patient, or other factors as determined by an operator of the device 10. For example, a high speed of gas 68 flow through the pores 66 of the membrane 60 will provide a very vigorous and turbulent environment in which a pathogen is very unlikely to settle and attach to the tips 64 of a structure 62. However, if an operator desires, the operator may slow the rate at which gas 68 is infused into the interior chamber 48, and will thus limit the amount of gas that is transferred into the vascular system of a patient. An operator may also prime the fluid line attached to the device 10 in order to remove any gas used or infused through the membrane 60. The device 10, or any device attached thereto, may also include a bubble trap, such as an IV filter, downstream of gas to trap gas bubbles prior to their entry into the vascular system of a patient. Thus, an operator may remove the gas before it enters into the vascular system of a patient.

The embodiments described with reference to Figures 4 through 9 may be modified to provide alternate flow of fluid and/or gas in order to provide a membrane capable of discouraging a pathogen from adhering to a vascular access device. For example, the fluid flow of the embodiments described with reference to Figures 4 through 7 may be reversed in order to provide a membrane that infuses both fluid and/or gas along with pathogens and/or biofilms off the interior surface 54 and into the interior chamber 48 and fluid path of the device 10. As another example, the embodiment described with reference to Figures 8 and 9 may be altered to provide an embodiment that reverses the flow of gas and/or fluid through the pores 66 of the membrane 60. In this alternate embodiment, the membrane 60 will pull and/or shear pathogens and/or biofilms into the pores 66 as a vacuum force is exerted upon them.
The scope of the invention is indicated by the appended claims, rather than by the foregoing description. All changes that come within the meaning and range of equivalency of the claims are to be embraced within their scope.

## Claims

1. A medical device, comprising: a vascular access device including a body (20) and a septum (22), wherein the septum communicates with a pathogenic environment, and the septum discourages biofilm formation, wherein the septum includes pores (34) and the vascular access device (10) includes a lubricant which is transferred across the septum through the pores, and wherein the septum is provided with a coating,
**characterized in that** said coating comprises a material capable of limiting the porosity of the septum, whereby controlling the rate at which lubricant is exuded to a surface of the septum that is mechanically attached or connected to the body (20).

2. The medical device of claim 1, wherein the septum (22) includes pores (34), wherein the vascular access device (10) includes an antimicrobial fluid (72), and wherein the antimicrobial fluid is transferred across the septum through the pores (34).

3. The medical device of claim 2, wherein the pores of the septum are canals (38).

4. The medical device of one of claims 1 - 3, wherein the vascular access device further includes a reservoir (36), and wherein the antimicrobial agent is housed within the reservoir.

5. The medical device of claim 2, wherein the antimicrobial agent is transferred as the vascular access device (10) is accessed by a separate device (26).

## Patentansprüche

1. Medizinische Vorrichtung mit: einer Gefäßzugangsvorrichtung mit einem Körper (20) und einem Septum (22), wobei das Septum mit einer pathogenen Umgebung in Verbindung steht, und das Septum die Bildung von Biofilm unterdrückt, wobei das Septum Poren (34) aufweist und die Gefäßzufuhrvorrichtung (10) ein Schmiermittel aufweist, das durch die Poren über das Septum transferiert wird, und wobei das Septum mit einer Beschichtung versehen ist,
**dadurch gekennzeichnet, dass** die Beschichtung ein Material aufweist, das in der Lage ist, die Porosität des Septums zu begrenzen, wodurch die Rate, mit welcher Schmiermittel auf die Oberfläche des Septums, das mechanisch an dem Körper (20) angebracht oder damit verbunden ist, abgegeben wird, geregelt wird.

2. Medizinische Vorrichtung nach Anspruch 1, bei welcher das Septum (22) Poren (34) aufweist, wobei die Gefäßzugangsvorrichtung (10) ein anti-mikrobielles Fluid (72) aufweist, und wobei das antimikrobielle Fluid durch die Poren (34) über das Septum transferiert wird.

3. Medizinische Vorrichtung nach Anspruch 2, bei welcher die Poren des Septums Kanäle (38) sind.

4. Medizinische Vorrichtung nach einem der Ansprüche 1 - 3, bei welcher die Gefäßzugangsvorrichtung ferner ein Reservoir (36) aufweist und wobei das antimikrobielle Mittel in dem Reservoir aufgenommen ist.

5. Medizinische Vorrichtung nach Anspruch 2, bei welcher das antimikrobielle Mittel transferiert wird, während der Zugang zu der Gefäßzugangsvorrichtung (10) mittels einer separaten Vorrichtung (26) erfolgt.

## Revendications

1. Dispositif médical comprenant: un dispositif d'accès vasculaire comprenant un corps (20) et un septum (22), dans lequel le septum communique avec un environnement pathogène, et le septum empêche la formation de biofilm, dans lequel le septum comprend des pores (34) et le dispositif d'accès vasculaire (10) comprend un lubrifiant qui est transféré sur le septum par les pores, et dans lequel le septum est doté d'un revêtement,
**caractérisé en ce que** ledit: revêtement comprend un matériau pouvant limiter la porosité du septum, régulant ainsi le débit auquel le lubrifiant est exsudé sur une surface du septum qui est mécaniquement fixée ou raccordée au corps (20).

2. Dispositif médical selon la revendication 1, dans lequel le septum (22) comprend des pores (34), dans lequel le dispositif d'accès vasculaire (10) comprend un fluide antimicrobien (72) et dans lequel le fluide antimicrobien est transféré sur le septum par les pores (34).

3. Dispositif médical selon la revendication 2, dans lequel les pores du septum sont des canaux (38).

4. Dispositif médical selon l'une des revendications 1 à 3, dans lequel le dispositif d'accès vasculaire comprend en outre un réservoir (36), et dans lequel l'agent antimicrobien est logé à l'intérieur du réservoir.

5. Dispositif médical selon la revendication 2, dans lequel l'agent antimicrobien est transféré au fur et à mesure qu'un dispositif séparé (26) a accès au dispositif d'accès vasculaire (10).
